# EUROPEAN PATENT SPECIFICATION

(11) **EP 3 316 878 B1**
(45) Date of publication and mention of the grant of the patent: **09.02.2022**
(21) Application number: 16739597.9
(22) Date of filing: 30.06.2016
(51) Int. Cl.: A61K 31/166, A61K 39/00, A61K 31/4184, A61K 31/47, G01N 33/00, A61P 19/02

(54) **METHODS AND COMPOUNDS FOR THE ALLEVIATION OF PAIN**
VERFAHREN UND VERBINDUNGEN ZUR LINDERUNG VON SCHMERZEN
PROCÉDÉS ET COMPOSÉS POUR LE SOULAGEMENT DE LA DOLOUR

(30) Priority: 03.07.2015 US 201562188499 P; 19.08.2015 US 201562206872 P
(43) Date of publication of application: 09.05.2018
(73) Proprietor: Catrina, Anca, 187 35 Täby (SE); Svensson, Camilla, 112 29 Stockholm (SE); Klareskog, Lars, 113 46 Stockholm (SE); Malmström, Vivianne, 116 43 Stockholm (SE)
(72) Inventor: Catrina, Anca, 187 35 Täby (SE); Svensson, Camilla, 112 29 Stockholm (SE); Klareskog, Lars, 113 46 Stockholm (SE); Malmström, Vivianne, 116 43 Stockholm (SE)
(74) Representative: Kransell & Wennborg KB
(86) International application number: PCT/SE2016/050673
(87) International publication number: WO 2017/007405

(56) References cited:
- WO-A1-2014/086365
- WO-A2-2011/050357
- US-A1- 2005 159 334
- US-A1- 2009 155 835
- V. C. WILLIS ET AL: "N- -Benzoyl-N5-(2-Chloro-1-Iminoethyl)-L-Orni thine Amide, a Protein Arginine Deiminase Inhibitor, Reduces the Severity of Murine Collagen-Induced Arthritis", THE JOURNAL OF IMMUNOLOGY, vol. 186, no. 7, 23 February 2011 (2011-02-23), pages 4396-4404, XP055308000, US ISSN: 0022-1767, DOI: 10.4049/jimmunol.1001620
- KRISHNAMURTHY, A. ET AL.: "A4.17 Anti-citrullinated proteins antibodies promotes osteoclastogenesis and bone destruction in rheumatoid arthritis", ANNALS OF THE RHEUMATIC DISEASES, vol. 74, no. Suppl 1, 13 February 2015 (2015-02-13), pages A43.2-A44, XP055308033, GB ISSN: 0003-4967, DOI: 10.1136/annrheumdis-2015-207259.100
- Ulrike Harre ET AL: "Induction of osteoclastogenesis and bone loss by human autoantibodies against citrullinated vimentin", The Journal of Clinical Investigation, vol. 122, no. 5, 1 May 2012 (2012-05-01), pages 1791-1802, XP55286894, GB ISSN: 0021-9738, DOI: 10.1172/JCI60975

## Description

### Technical field

The present description relates generally to the alleviation, treatment and/or prevention of pain in subjects exhibiting elevated activation of osteoclasts, in particular in subjects also exhibiting autoantibodies, individuals at risk of developing disease and exhibiting autoantibodies, and in particular subjects exhibiting antibodies against citrullinated protein/peptide antigens.

### Background

Rheumatoid arthritis (RA) is a chronic inflammatory joint disease. Antibodies against citrullinated protein/peptide antigens (ACPAs) occur in a majority of patients and are highly specific for RA. ACPAs consist of a collection of antibodies with different specificities toward citrullinated antigens. It is generally known that ACPAs may occur many years before the onset of joint inflammation, and their presence has been associated with bone destruction (Rantapää-Dahlqvist et al., A&R 2003; Harre et al., JCI 2012).

Citrullination is a post-translational modification where arginine (Arg) is converted to citrulline (Cit) by an enzymatic reaction catalyzed by peptidylarginine deiminases (PAD). *In vitro* activation of PAD enzymes is known to require high levels of calcium (Takahara et al.,1986; Mechin et al., 2005; and Vossenaar et al., 2003).

Citrullination was originally described as a physiological process in the terminal differentiation of the epidermis and during brain development, but is also shown to be a central event in the context of inflammation (Makrygiannakis et al., 2006; and Vossenaar et al., 2004).

Bone destruction is largely dependent on bone resorption by osteoclasts (OCs), multinucleated giant cells that originate from either macrophages (MΦ) or immature dendritic cells (iDC) in the presence of RANKL and M-CSF (Teitelbaum, 2000).

Bone destruction is a hallmark of rheumatoid arthritis, classically believed to reflect only the inflammatory burden in joints; however, bone destruction may occur despite inactive disease (Molenaar *et al.,* 2004) and even in the absence of detectable inflammation in the joints of ACPA-positive individuals at risk of developing RA (Kleyer *et al.,* 2013) who do not yet have the disease but might have joint pain.

One potential explanation for these observations is the recently described direct effect of ACPAs on bone metabolism. In the report from Harre et al., polyclonal ACPAs isolated from the peripheral blood (PB) of RA patients purified on an affinity column with mutated citrullinated vimentin (MCV), were shown to promote bone resorption *in vitro* through a tumor necrosis factor (TNF)-mediated mechanism and to induce osteoclastogenesis by adoptive transfer into mice (Harre, *et al.* 2012).

One aim of the present study was to better understand ACPAs' effects on OCs and to develop new approaches to alleviation, treatment and/or prevention of bone loss and/or pain in subjects exhibiting elevated activation of osteoclasts, in particular in subjects also exhibiting autoantibodies, individuals at risk of developing disease and exhibiting autoantibodies, and in particular subjects exhibiting antibodies against-citrullinated protein/peptide antigens. An additional aim has been to understand the role of OC:s in the initiation and propagation of arthritis, in particular in individuals exhibiting antibodies against citrullinated antigens.

In particular, the inventors set out to analyze which mediators may be produced by OCs after exposure to ACPAs and how such mediators may be related to the development of joint inflammation and bone destruction. Furthermore, the inventors investigated whether OCs differentiation and effector functions are dependent on citrullination. To address these questions, the inventors used multiple methods of inducing OCs and different polyclonal ACPAs, which were affinity-purified from either synovial fluid (SF) and/or peripheral blood (PB) from patients with ACPA-positive RA. The inventors also used human monoclonal ACPAs with varying fine specificities, which were generated from joint-derived single B cells/plasma cells of RA patients.

Citrullination is a common feature of inflammation. The presence of anti-citrullinated protein/peptide antibodies (ACPA), however, is unique to rheumatoid arthritis. Several lines of evidence suggest that ACPA are important in the pathogenesis of rheumatoid arthritis. A relevant hypothesis for this pathogenesis is a two-hit model. The first hit gives rise to ACPA, and the second hit, an unrelated episode of synovial inflammation accompanied by citrullination, is perpetuated by the pre-existing antibodies. This model suggests that reducing citrullination might ameliorate disease.

WO2014086365A1 - This international application relates to anti-peptidylarginine deiminase 2 (PAD2) antibodies and anti-PAD2 antibodies for use in the treatment of autoimmune diseases characterized by extracellular citrullination, such as rheumatoid arthritis (RA). The application further relates to a method for treatment of an autoimmune disease characterized by extracellular citrullination comprising the administration of a suitable amount of an anti-PAD2 antibody to a subject. The alleviation of pain is not mentioned, and also not any prevention of bone destruction.

US20050159334A1 - This US application discloses the treatment of RA with the administration of a therapeutic dose of a therapeutically acceptable PAD inhibitor. Administration can occur after the onset of RA symptoms, or prophylactically before such symptoms present. In one embodiment, the inhibitor has a side chain including a benzamide group to the left and an ester group to the right of a peptide bond. Bone destruction is not addressed, nor is the alleviation of pain.

US8338188B2 - This US patent relates to the identification and use of proteins with clinical relevance to rheumatoid arthritis (RA). In particular, the invention provides the identity of marker proteins that specifically react with RA-associated autoantibodies. Also provided are methods, arrays and kits for using these proteins in the diagnosis of RA, and in the selection and/or monitoring of treatment regimens. The patent also comprises detecting anti-PAD4 antibodies in a biological sample obtained from a subject suspected of having RA.

### Summary

This disclosure relates to compounds for use in alleviating pain in a subject wherein said pain is associated with an elevated activation of osteoclasts in said subject, wherein said elevated activation of osteoclasts is associated with the presence of anti-citrullinated protein antibodies (ACPA) in said subject, said autoantibodies are detectable in a sample taken from a subject or patient, but said subject does not manifest clinical signs of rheumatoid arthritis, and wherein an effective amount of a compound capable of inhibiting the activity of peptidylarginine deiminase (PAD) enzymes is administered to said subject.

According to an embodiment of said third aspect, said compound is an amidine compound.

Preferably said amidine compound is chosen from the compounds exemplified in Table 1 below:

| **Table 1. Examples of amidine derived PAD-inhibitors** | |
|---|---|
| Name: | Formal name: |
| F-amidine | N-[(1S)-1-(aminocarbonyl)-4-[(2-fluoro-1-iminoethyl)amino]butyl]-2,2,2-trifluoroacetate-benzamide |
| Cl-amidine | N-α-benzoyl-N5-(2-chloro-1-iminoethyl)-L-Orn amide |
| BB-CI-amidine | N-[(1S)-1-(1H-benzimidazol-2-yl)-4-[(2-chloro-1-iminoethyl)amino]butyl]-[1,1'-biphenyl]-4-carboxamide |
| TDFA | Thr-Asp-F-amidine |
| BTT-Cl-amidine | Biphenyl tetrazole tert-butyl Cl-amidine |
| o-F-amidine | N-α-(2-carboxyl)benzoyl-N(5)-(2-fluoro-1-iminoethyl)-l-ornithine amide |
| o-Cl-amidine | N-α-(2-carboxyl)benzoyl-N(5)-(2-chloro-1-iminoethyl)-l-ornithine amide |

According to a preferred embodiment of said third aspect, said compound is streptonigrin (SID 11532976).

The present inventors contemplate that the effects of ACPAs or other autoantibodies may be further enhanced by the presence of rheumatoid factors (RF).

The present disclosure thus concerns the use of a PAD inhibitor for the alleviation and/or prevention of pain associated with an elevated activation of osteoclasts in a subject, wherein the elevated activation of osteoclasts is associated with the presence of anti-citrullinated protein antibodies (ACPA) in the subject. Said autoantibodies may comprise or consist predominantly of anti-citrullinated protein antibodies (ACPA) and/or antibodies cross-reacting with targets of ACPAs. More preferably, said autoantibodies are anti-citrullinated protein antibodies (ACPA). The present inventors contemplate that the effects of ACPAs or other autoantibodies may be further enhanced by the presence of rheumatoid factors (RF).

Preferably said compound is an amidine compound. More preferably said amidine compound is chosen from compounds exemplified in Table 1.

According to an embodiment, said compound is streptonigrin (SID 11532976). According to another embodiment, said compound is an 1,2,3-triazole peptidomimetic-based derivative. According to yet another embodiment, said compound is an anti-peptidylarginine deiminase (PAD) antibody.

According to a preferred embodiment, freely combinable with the above, said autoantibodies are detectable in a sample taken from said subject or patient, but wherein said subject does not manifest clinical signs of rheumatoid arthritis. In this embodiment, said autoantibodies may comprise or consist predominantly of anti-citrullinated protein antibodies (ACPA) and/or antibodies cross-reacting with targets of ACPAs. More preferably, said autoantibodies are anti-citrullinated protein antibodies (ACPA). As stated above, the present inventors contemplate that the effects of ACPAs or other autoantibodies may be further enhanced by the presence of rheumatoid factors (RF).

Another aspect relates to a diagnostic kit for use in identifying individuals that would benefit from a method of alleviating pain in a subject wherein said pain is associated with an elevated activation of osteoclasts in said subject and wherein said elevated activation of osteoclasts is associated with the presence of autoantibodies in said subject, wherein said method and/or kit comprises one or more of the following method steps or components:
- an assay for determining the level osteoclast activation,
- an assay for determining the presence and identity of autoantibodies, including presence of antibodies to citrullinated antigens and/or the presence of rheumatoid factors (RF), and
- a questionnaire for quantitatively and optionally qualitatively assessing pain, and in particular joint pain (arthralgia), and optionally also
- an assay, the means for, or a step of qualitatively or quantitatively assessing bone density, e.g. the degree of bone loss, for example means relying on the use of ultrasound, dual X-ray absorptiometry (DXA), dual energy X-ray absorptiometry (DEXA), or a special X-ray called quantitative computed tomography (QCT).

Yet another aspect, not claimed, concerns methods for identifying compounds effective to alleviate bone loss and/or pain, wherein said compounds are evaluated based on their capability to inhibiting or blocking the activation of osteoclasts.

Further aspects and embodiments will become apparent to a person skilled in the art upon study of the figures and the following detailed description and examples.

### Brief description of drawings

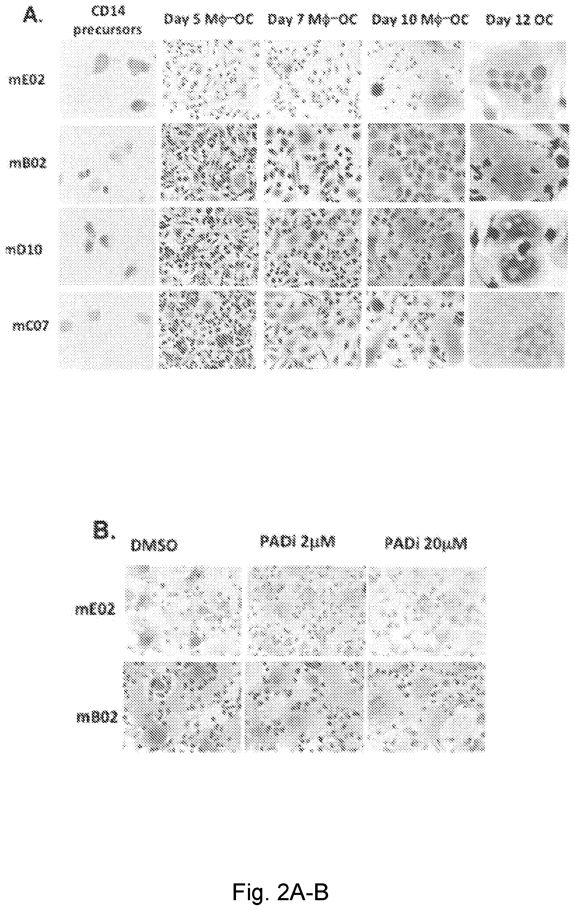

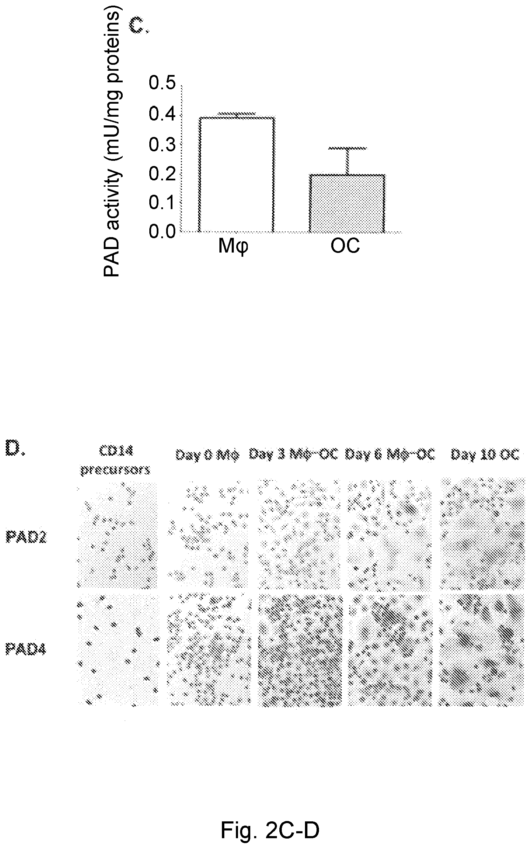

The invention is now described, by way of example, with reference to the accompanying drawings, in which:

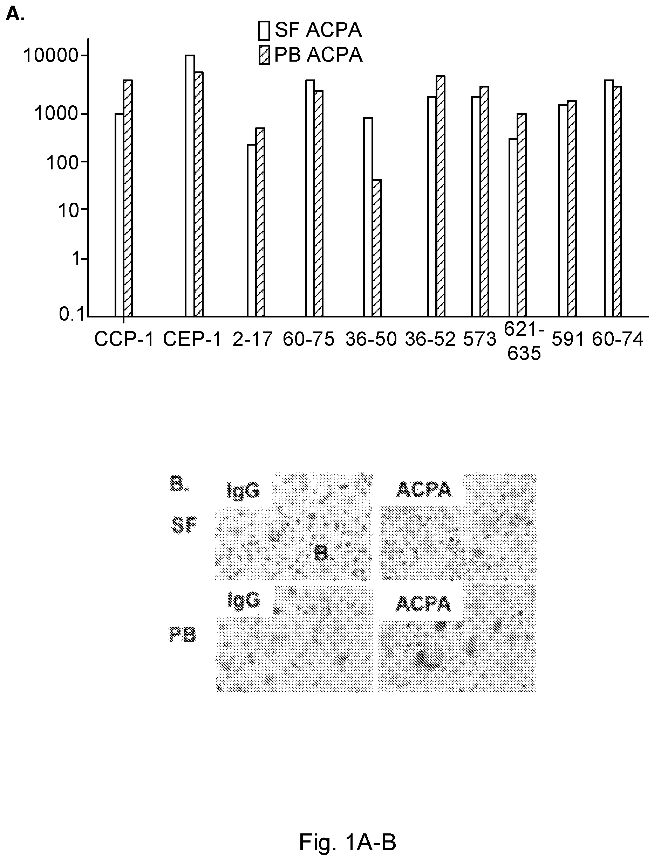

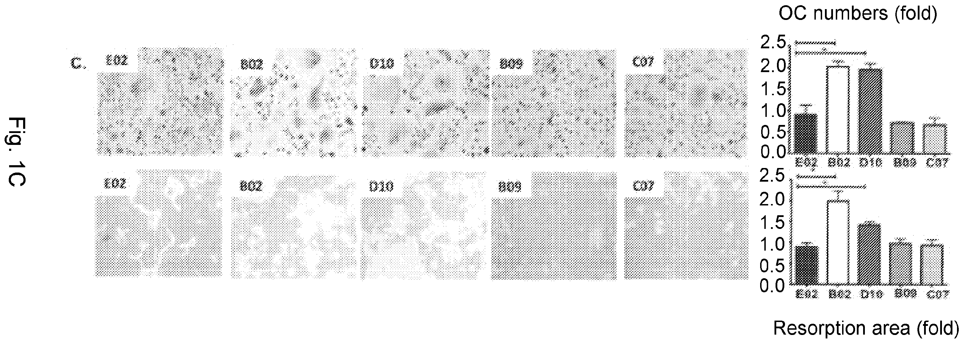

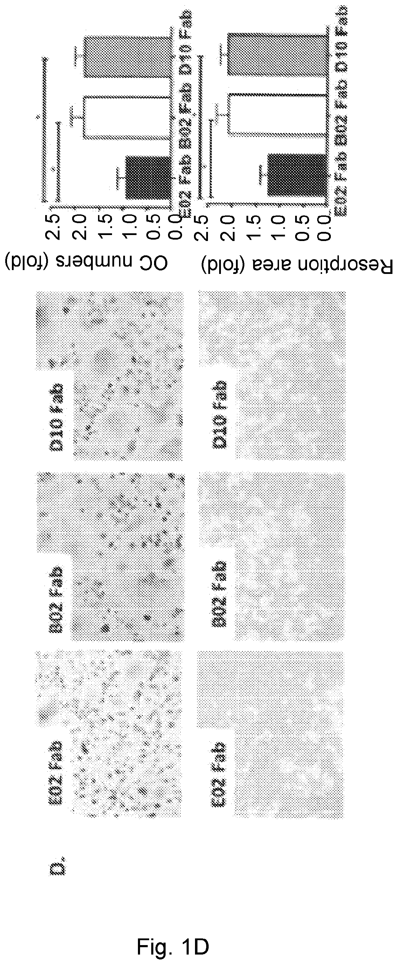

**Figure 1** illustrates that polyclonal (anti CCP-2 affinity-purified) and monoclonal (single B cell-derived) ACPAs induce osteoclast activation and bone resorption:
   **A.** Multiplex chip-based assay results showing that PB and SF ACPA pools contain a wide spectrum of human ACPAs with reactivity against multiple citrullinated targets; values are expressed as arbitrary units/ml.
   **B.** TRAP staining of mature OCs obtained from Mφ derived from CD14-positive monocytes of healthy individuals and cultured in the presence of either non-ACPA flow-through IgGs (IgG) or ACPA IgGs (ACPA) at a concentration of 0.1 µg/ml (original magnification 200X). The graph represents the fold increase in OC (TRAP positive cells with ≥3 nuclei) numbers and fold increase in resorption areas. The values represent the mean±SEM of 3 independent experiments.
   **C.** TRAP staining of mature OCs and microscopic visualization of calcium phosphate resorption areas in the presence of 4 monoclonal ACPAs (i.e., B02, D10, B09 and C07) and one control anti-tetanus monoclonal antibody (i.e., E02) at a concentration of 1 µg/ml. The graphs represent fold increases in OC (TRAP-positive cells with ≥3 nuclei) numbers and fold increases in resorption area. The values represent the mean±SEM of 4 independent experiments.
   D. TRAP staining of mature OCs and microscopic visualization of calcium phosphate resorption area in the presence of Fab fragments of D10, B02 and E02 antibodies (1 µg/ml). (N=4). The graphs represent fold increases in OC (TRAP positive cells with ≥3 nuclei) numbers and fold increases in resorption area. The values represent the mean±SEM of 4 independent experiments. *p<0.05
**Figure 2** illustrates the expression of citrullinated targets and PAD enzymes during different stages of OC differentiation.
   **A.** Immunohistochemistry images showing brown diaminobenzidine (DAB) staining of citrullinated targets in different stages of differentiation from CD14-positive monocyte precursors to Mφ and mature OCs. Slides were stained with murinized monoclonal ACPAs (mB02, mD10, mC07) and a monoclonal control antibody (mE02) and counterstained with hematoxylin (original magnification 500X for CD14-positive monocytes and mature OCs and 250X for the intermediate stages).
   **B.** Immunohistochemistry images showing brown diaminobenzidine (DAB) staining of citrullinated targets in mature OCs with or without incubation with a PAD inhibitor (Cl-amidine) added from the beginning of the cultures. Slides were stained with murinized monoclonal ACPAs (mB02) and a monoclonal control antibody (mE02) and counterstained with hematoxylin (original magnification 250X).
   **C.** PAD activity was measured using an antibody-based assay by adding MΦ and OC cell lysates to arginine-coated plates, followed by ELISA measurement of the amounts of deiminated arginine. The graph represents the PAD enzyme activity expressed in mU/mg protein. The values represent the mean±SEM of two independent experiments.
   **D.** Immunohistochemistry images showing brown diaminobenzidine (DAB) staining of PAD2 and PAD4 expression in different stages of differentiation from CD-14-positive monocyte precursors to Mφ and mature OCs. Slides were counterstained with hematoxylin (original magnification 250X).

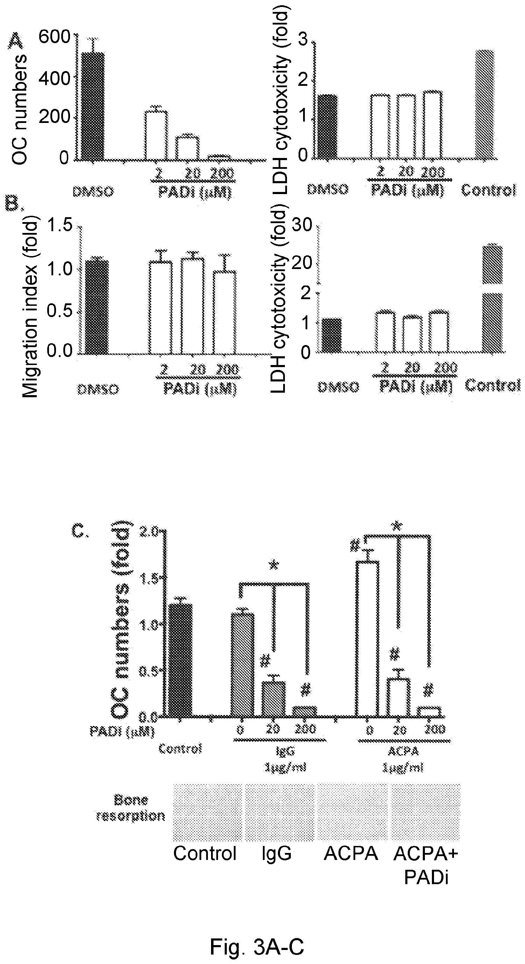

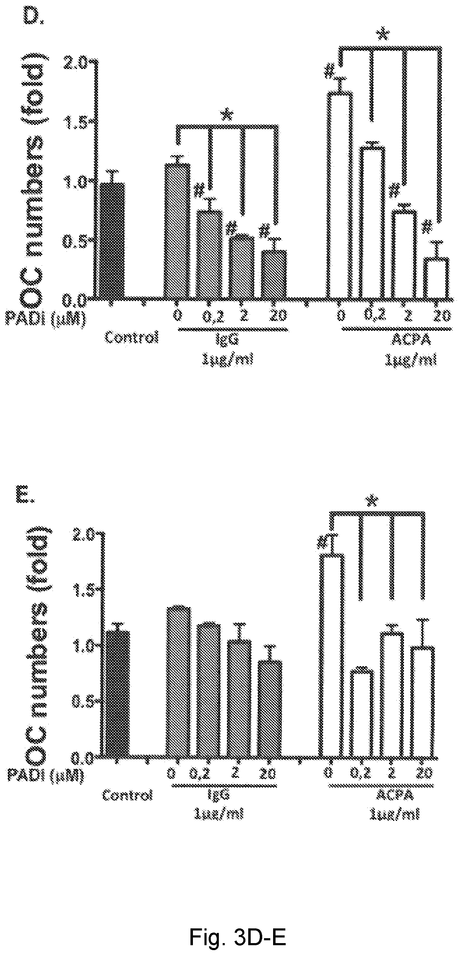

**Figure 3** shows that PAD enzymes are essential for osteoclastogenesis and the ACPA-mediated effect.
   **A.** PAD inhibition (PADi, Cl-amidine) dose-dependently inhibited OC differentiation and maturation without any cytotoxic effect. The graphs represent fold decreases in OC (TRAP-positive cells with ≥3 nuclei) numbers and fold increases in LDH release in the culture supernatants. The values represent the mean±SEM.
   **B.** PADi does not affect either SF migration or survival. The graphs represent fold increases in the migration index of synovial fibroblast and LDH release in the culture supernatants. The values represent the mean±SEM.
   **C.** The addition of PADi from the beginning of the OC cultures prevented ACPA-induced OC activation and calcium phosphate resorption. The graphs represent fold increases in OC (TRAP-positive cells with ≥3 nuclei) numbers. The values represent the mean±SEM of 3 independent experiments. Images represent the resorption area by OCs (original magnification 40X).
   **D.** Dose titration of PADi showing that early PAD inhibition (at the initiation of the OC culture) with doses as low as 0.2 µM PADi inhibits ACPA-mediated osteoclastogenesis but no longer the unstimulated differentiation of OCs. The graphs represent fold decreases in OC (TRAP-positive cells with ≥3 nuclei) numbers. The values represent the mean±SEM.
   **E.** Late PAD inhibition (3 days before ending the OC cultures) inhibited ACPA-mediated osteoclastogenesis but not the unstimulated differentiation of OCs. The graphs represent fold increases in OC (TRAP-positive cells with ≥3 nuclei) numbers. The values represent the mean±SEM. *p<0.05.

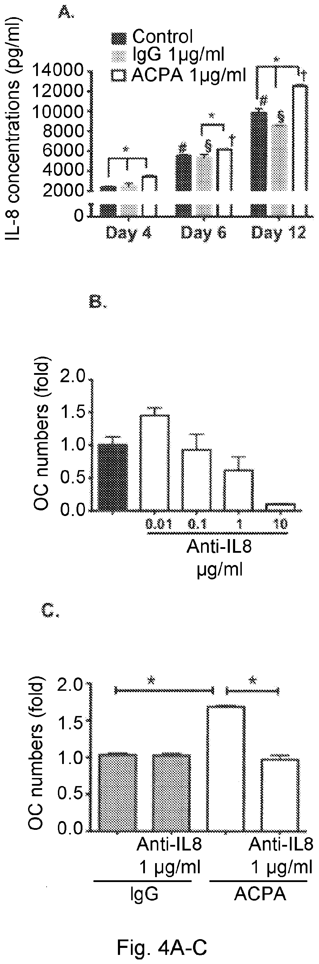

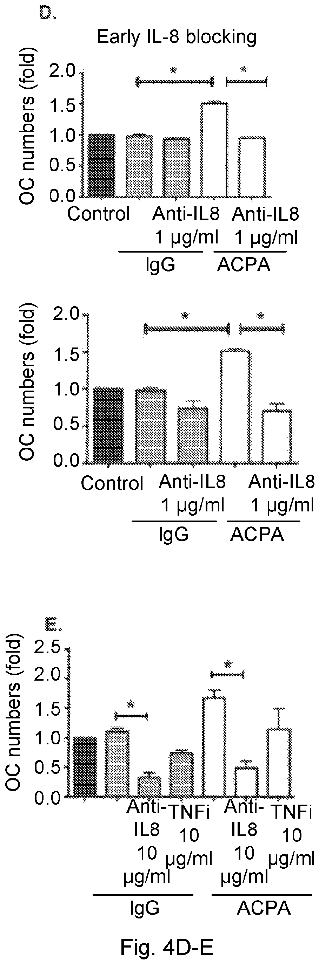

**Figure 4** shows that IL-8 is an essential mediator of ACPA-driven osteoclastogenesis.
   **A.** Cytometric bead array showed high levels of IL-8 in M□-derived OC cultures at early time points during their maturation, which further increased over time. ACPA, but not control IgGs, additionally increased IL-8 release in the culture supernatants at all time points tested. The graph shows a representative time kinetic variation in IL-8 concentrations in cell culture supernatants from one of the three tested donors. The values represent the mean±SEM.
   **B.** Neutralizing anti-IL-8 antibodies inhibited MΦ-derived OCs maturation dose dependently. The graphs represent fold decreases in OC (TRAP-positive cells with ≥3 nuclei) numbers. The values represent the mean±SEM of 3 independent experiments.
   **C.** Anti-IL-8 neutralizing antibodies completely abolished the effect of ACPAs at doses as low as 1 µg/ml. The graphs represent fold increases in OC (TRAP-positive cells with ≥3 nuclei) numbers. The values represent the mean±SEM of 3 independent experiments.
   **D.** Both early (first 3 days of culture) and late (last 3 days of the culture) addition of anti-IL-8 neutralizing antibodies (1 µg/ml) completely abolished the effect of ACPAs. The graphs represent fold increases in OC (TRAP-positive cells with ≥3 nuclei) numbers. The values represent the mean±SEM.
   **E.** Anti-IL-8 neutralizing antibodies but not an antibody against TNF-a (adalimumab) abolished the effect of ACPAs at concentrations as high 10 µg/ml. The graphs represent fold increases in OC (TRAP-positive cells with ≥3 nuclei) numbers. The values represent the mean±SEM of 3 independent experiments. *p<0.05.

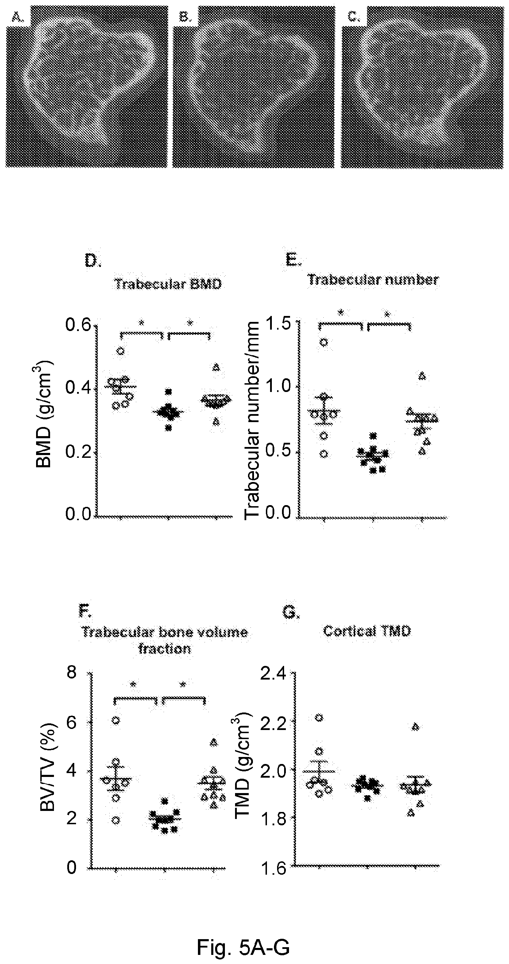

**Figure 5** shows that ACPAs induce systemic bone loss *in vivo* that is reversed by IL-8 inhibition.
   Representative two dimensional micro-computer tomography images of the tibial metaphysis of control mice (A, n=7) and mice that were injected with ACPAs in the absence (B, n=9) or presence of reparixin (C, n=9).
   The graphs D, E, F and G show the results of a quantitative evaluation of the trabecular bone mineral density (BMD, D), trabecular number (E), bone volume fraction (bone volume/tissue volume, F) and the cortical tissue mineral density (TMD, G). The values represent the mean±SEM. *p<0.05.

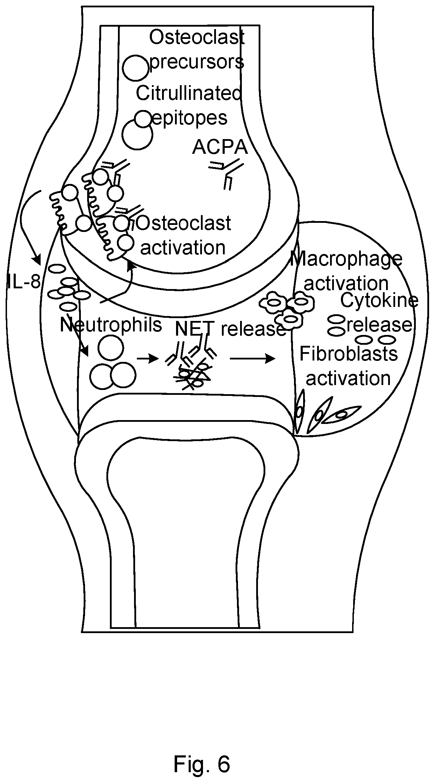

**Figure 6** is a schematic illustration of the PAD-dependent differentiation and maturation of OCs, allowing initial OC targeting by ACPAs with consecutive IL-8 release.
   The figure shows how OC precursors (OCPs) are present in the bone marrow and can develop into mature OCs. During the differentiation and activation of OCP, a gradual increase in cell citrullination occurred as a consequence of increased PAD activity in a calcium-rich microenvironment. ACPAs present in the circulation can reach and bind to maturing OCPs in the bone marrow, leading to an increase in OC activity with consecutive bone resorption through an IL-8-dependent autocrine loop. In a second step, IL-8 will reach the joint and initiate the chemoattraction and migration of inflammatory cells in particular neutrophils. Neutrophil extracellular traps are released by these neutrophils in the presence of ACPAs, which further contributes to the initiation of joint inflammation with the local accumulation of other inflammatory cells (such as macrophages) and activation of synovial fibroblasts, resulting in synovial membrane inflammation.
**Figure 7** shows the effect of PAD-inhibition using BB-CI-amidine expressed as osteoclast number (A) estimated with tartrate-resistant acid phosphatase (TRAP) staining, bone resorption (B) determined as erosion (%), and cytotoxicity (C) determined using for the cell counting kit 8 (CCK-8) for the quantitation of viable cell number.
**Figure 8** shows the effect of different concentrations of reparixin on osteoclast number (A), and Reparixin L-lysine salt on osteoclast number and cytotoxicity (graphs B and C, respectively) *in vitro.*
**Figure 9** shows the effect of different concentrations of SCH 527123 on osteoclast number and cytotoxicity measured as optical density at 450 nm (OD 450) (graphs A and B, respectively) *in vitro.*
**Figure 10** shows the effect of different concentrations of SB 332235 on osteoclast number and cytotoxicity measured as optical density at 450 nm (OD 450) (graphs A and B, respectively) *in vitro*

### Description of embodiments

Before the present invention is described, it is to be understood that the terminology employed herein is used for the purpose of describing particular embodiments only and is not intended to be limiting, since the scope of the present invention will be limited only by the appended claims.

It must be noted that, as used in this specification and the appended claims, the singular forms "a," "an," and "the" include plural referents unless the context clearly dictates otherwise.

The terms "treatment", "therapy", "therapeutic use", "medicament", and "medical use" encompass both human and animal or veterinary applications.

The term "elevated" as in "elevated activation of osteoclasts" is used to indicate a level discernably higher than the level of activation typical for a healthy individual, or higher than a level previously measured for the same individual, or higher than an average level for healthy individuals. A person skilled in the art will understand the meaning of the term "elevated" as such as person, e.g. a physician, is well familiar with features characteristic for a general, healthy population, for different populations, and for subjects suffering from a disease but with different severity. Such a skilled person will recognize when a feature deviates, and it is immediately recognized if this deviation represents an increased or elevated value, or a reduced, lowered value.

The terms "contribute" and "contribution" as in "... increased activation of osteoclasts contribute to the bone loss" and "bone loss associated with the contribution of antibodies" and other expressions in this description and claims, are intended to cover all interaction and dependencies between for example osteoclasts or antibodies, and detectable bone loss.

The terms "inhibit", "inhibition" or "blockade" are used to describe an inhibition of a significant part of the action of peptidyl arginine deiminase enzymes (PAD) and the activation of osteoclasts, distinguished from a total blocking of this action. It is contemplated that an inhibition or blockade of the action of PAD is preferable to a total blocking of the same, considering that PAD enzymes have many functions in the mammalian organism.

As briefly summarized above, the present description concerns methods and compounds for the alleviation of pain in conditions where an increased activation of osteoclasts contribute to the bone loss and/or pain, in other words where there is an action or effect of the activation of osteoclasts in a subject, i.e. in situations where osteoclasts contribute to the pain.

One group of diseases exhibiting both these features are autoimmune diseases, in which both pain and bone loss are serious consequences of the disease. Rheumatoid arthritis, osteoarthritis and arthralgias of different etiology can be mentioned as examples.

Bone loss occurs also in other diseases and as a result of different conditions, such as autoimmune diseases, e.g. rheumatoid arthritis, lupus, multiple sclerosis, and ankylosing spondylitis; as a consequence of gastrointestinal disorders, e.g. vitamin deficiencies, celiac disease, Crohn's disease and ulcerative colitis; gastrointestinal bypass procedures; endocrine and hormonal disorders, e.g. hyperparathyroidism, hyperthyroidism, diabetes, disorders reflected as deviations in testosterone and/or estrogen levels; hematologic disorders, e.g. leukemia, multiple myeloma, different cancers, including metastases to bone, sickle cell disease; AIDS/HIV, and other chronic diseases.

However, in many cases, bone loss is not a symptom of the disease itself, but rather a side-effect of the disease such as malnutrition or disturbed hormonal levels, or it can even be a side-effect of the medication, for example a side-effect of androgen deprivation therapy in the treatment of prostate cancer, or a side-effect of steroid medications in the treatment of autoimmune diseases.

One example of bone loss is periodontitis, which can be caused by infection and/or inflammation in the gums, tumors in the jaws, as a result of general osteoporosis, or as a side-effect of medication or nutritional deficiencies as exemplified above.

A third aspect is a method of preventing and/or alleviating pain in a subject wherein said pain is associated with an elevated activation of osteoclasts in said subject, wherein an effective amount of a compound capable of inhibiting the activity of peptidylarginine deiminase (PAD) enzymes is administered to said subject.

According to an embodiment of said third aspect, said compound is an amidine compound. Preferably said amidine compound is chosen from the compounds exemplified in Table 1.

| **Table 1. Examples of amidine derived PAD-inhibitors** | |
|---|---|
| Name: | Formal name: |
| F-amidine | N-[(1S)-1-(aminocarbonyl)-4-[(2-fluoro-1-iminoethyl)amino]butyl]-2,2,2-trifluoroacetate-benzamide |
| Cl-amidine | N-α-benzoyl-N5-(2-chloro-1-iminoethyl)-L-Orn amide |
| BB-CI-amidine | N-[(1S)-1-(1H-benzimidazol-2-yl)-4-[(2-chloro-1-iminoethyl)amino]butyl]-[1,1'-biphenyl]-4-carboxamide |
| TDFA | Thr-Asp-F-amidine |
| BTT-Cl-amidine | Biphenyl tetrazole tert-butyl Cl-amidine |
| o-F-amidine | N-α-(2-carboxyl)benzoyl-N(5)-(2-fluoro-1-iminoethyl)-l-ornithine amide |
| o-CI-amidine | N-α-(2-carboxyl)benzoyl-N(5)-(2-chloro-1-iminoethyl)-l-ornithine amide |

According to a preferred embodiment of said third aspect, said compound is streptonigrin (SID 11532976).

Another aspect - not claimed - is a method of preventing and/or alleviating pain in a subject wherein said pain is associated with an elevated activation of osteoclasts in said subject, wherein the elevated activation of osteoclasts is associated with the presence of autoantibodies in said subject, characterized in that an effective amount of a compound capable of inhibiting the activity of peptidylarginine deiminase (PAD) enzymes is administered to said subject.

According to a preferred embodiment of said aspect, said autoantibodies are anti-citrullinated protein antibodies (ACPA). In this embodiment, said autoantibodies may comprise or consist predominantly of anti-citrullinated protein antibodies (ACPA) and/or antibodies cross-reacting with targets of ACPAs. More preferably, said autoantibodies are anti-citrullinated protein antibodies (ACPA). As stated above, the present inventors contemplate that the effects of ACPAs or other autoantibodies may be further enhanced by the presence of rheumatoid factors (RF).

According to a preferred embodiment, said autoantibodies are detectable in a sample taken from said patient, but the patient does not manifest clinical signs of an autoimmune disease.

According to an embodiment, freely combinable with the above, said autoimmune disease is chosen from rheumatoid arthritis, osteoarthritis, and arthralgia.

The invention makes available the use of PAD inhibitor for the alleviation of pain associated with an elevated activation of osteoclasts in a subject, wherein said elevated activation of osteoclasts is associated with the presence of anti-citrullinated protein antibodies (ACPA) in the subject, and wherein said anti-citrullinated protein antibodies (ACPA) are detectable in a sample taken from said subject, but wherein the subject does not manifest clinical signs of rheumatoid arthritis. In this embodiment, said autoantibodies may comprise or consist predominantly of anti-citrullinated protein antibodies (ACPA) and/or antibodies cross-reacting with targets of ACPAs. More preferably, said autoantibodies are anti-citrullinated protein antibodies (ACPA). As stated above, the present inventors contemplate that the effects of ACPAs or other autoantibodies may be further enhanced by the presence of rheumatoid factors (RF).

In the use according to the above, said compound is an amidine compound. Preferably said amidine compound is chosen from the compounds exemplified in Table 1.

According to an embodiment, said compound is streptonigrin (SID 11532976). According to an embodiment, freely combinable with any of the aspects and embodiments presented herein, the above mentioned compound or combination of compounds is administered systemically. Systemic administration includes enteral and parenteral routes of administration, well known to persons skilled in the art. Examples of enteral routes of administration include oral, rectal and sublingual administration. Examples of parenteral routes of administration include intravenous, intramuscular, and subcutaneous administration. Other routes of administration, suitable depending on the composition of the final drug based on the findings in this disclosure, include intra-articular, topical, transdermal, nasal, intratracheal, intraventricular, and intrapulmonar administration.

According to a preferred embodiment, freely combinable with the above, said autoantibodies are detectable in a sample taken from said patient, but wherein said patient does not manifest clinical signs of an autoimmune disease. In this embodiment, said autoantibodies may comprise or consist predominantly of anti-citrullinated protein antibodies (ACPA) and/or antibodies cross-reacting with targets of ACPAs. More preferably, said autoantibodies are anti-citrullinated protein antibodies (ACPA). As stated above, the present inventors contemplate that the effects of ACPAs or other autoantibodies may be further enhanced by the presence of rheumatoid factors (RF).

Methods and assays for the determination osteoclast activation are available. The present inventors evaluated osteoclast activity by measuring the resorption area under low magnification using NIS elements software (Nikon Instruments Europe BV, Amsterdam, Netherlands) as disclosed in the examples.

Assays for the qualitative and quantitative analysis of antibodies are also available, for example the cyclic citrullinated peptide (CCP) antibody test. One commercially available CCP test is the Immunoscan CCPlus^{®}, supplied by Euro Diagnostica AB, Malmö, Sweden. This is an enzyme-linked immunosorbent assay (ELISA) for qualitative and semi-quantitative determination of IgG antibodies to Cyclic Citrullinated Peptides (CCP) in human sera. This assay recognizes both antibodies (ACPAs) able to activate osteoclasts and induce IL-8 production and other ACPAs not able to activate osteoclasts and induce II-8 production. Therefore this assay is useful but not optimal for identifying subjects or patients at risk of developing pain and/or bone loss as well as at risk of developing RA or other autoimmune disease. The inventors are currently using a modified high sensitivity and fine specificity ACPA test based on a multiplex fluorescent detection assay which enables the inventors to specifically identify specific anticitrulline antibodies (ACPAs) with potentials to active osteoclasts, and to induce IL-8 production from osteoclasts, and to cause pain that is dependent on production of IL-8 from osteoclasts.

Similarly, the inventors are currently using a questionnaire and visual pain assessment tool.

Another aspect relates to a diagnostic kit for use in identifying individuals that would benefit from the above mentioned treatment, the alleviation or prevention of pain, wherein said method and/or kit comprises one or more of the following:
- an assay for determining the level osteoclast activation,
- an assay for determining the presence and identity of autoantibodies, including presence of antibodies to citrullinated antigens and/or the presence of rheumatoid factors (RF), and
- a questionnaire for quantitatively and optionally qualitatively assessing pain, and in particular joint pain (arthralgia).

Another aspect -not claimed - is a method for identifying compounds effective to alleviate bone loss and/or pain, wherein said compounds are evaluated based on their capability to inhibiting or blocking the activation of osteoclasts

### Examples

### Material and methods

### Patients

RA patients attending the Rheumatology Clinic at Karolinska University Hospital and fulfilling the 1987 American College of Rheumatology criteria for the diagnosis of RA were included in the study. Informed consent was obtained from all patients in accordance with a protocol approved by the Ethical Review Committee North of Karolinska University Hospital. Non-paired SF (n=26) and plasma (n=38) samples were collected from ACPA+ RA patients for polyclonal ACPA isolation. SF samples from three ACPA-positive RA patients (3 females with a median age of 37 years, range 37-47) and one ACPA-negative RA patient were used for the generation of monoclonal ACPAs (B02, D10, B09 and C07) and control E02 anti tetanus toxoid antibody (male, 36 years old). Fresh blood samples from either the blood donor buffy coats or the peripheral blood of ACPA-positive RA patients (n=4, 3 females and 1 males, median age 51, range 44-75) were also collected and used for monocyte isolation and OC generation.

### ACPA generation

Total IgGs from the SF and plasma of RA patients were isolated on Protein G followed by ACPA IgG affinity purification on CCP2 columns as described previously (Ossipova, et al., 2014). Monoclonal ACPAs RA1103:01:B02 (B02), RA1276:01:D10 (D10), RA 1325:01:B09 (B09) and RA1276:01:C07 (C07) and monoclonal anti tetanus toxoid antigen control monoclonal antibody RA1362:01:E02 (E02) were isolated from single B-cells isolated from SF of ACPA-positive RA patients as previously described (Amara et al., 2013). Monomeric Fab fragments of B02, D10 and E02 monoclonal antibodies were obtained using the same methodology. All of the monoclonal antibodies were tested at concentrations of 1 µg/ml. The Fc part was exchanged for a murine IgG2a Fc part to generate murinized mE02, mB02, mD10 and mC07 (Amara et al., 2013) for use in immunohistochemistry. All of the antibody preparations were endotoxin free.

### Osteoclast cultures

Monocytes were isolated through a Ficoll preparation (Lymphoprep; Axis Shield, Norway), followed by positive selection with anti CD-14 conjugated microbeads (Miltenyi Biotec Norden, Lund, Sweden). Mϕ were generated by directly seeding CD14+ monocytes at 105 cells/well in 96-well plates in DMEM medium containing 10% heat inactivated Fetal Bovine Serum (FBS), 2 mM L-glutamine, 100 IU/ml Penicillin and 50 µg/ml streptomycin along with M-CSF at 25 ng/ml for 3 days. iDCs were generated from CD14+ monocytes seeded at 106 cells/ml in a six-well plate with RPMI medium containing 10% heat inactivated FBS, 2 mM L-glutamine, 100 IU/ml penicillin and 50 µg/ml streptomycin along with cytokines GM-CSF at 75 ng/ml and IL-4 at 50 ng/ml for six days iDCs were generated from CD14+ monocytes (Nasi *et al.,* 2013). RANKL was obtained from R&D Systems, Abingdon, UK; GM-CSF, IL-4 and M-CSF were ordered from Peprotech, London, UK. All of the other cell culture reagents were purchased from Sigma-Aldrich, Stockholm, Sweden.

OCs were developed from either Mϕ or iDC in the presence of M-CSF (concentration range 10-30 ng/ml) and RANKL (concentrations range 2.5-5 ng/ml) with or without polyclonal ACPA, control polyclonal IgGs, monoclonal ACPAs or control monoclonal antibody. The medium was exchanged every three days. At the end of the culture, the OCs were analyzed using tartrate-resistant acid phosphatase (TRAP) staining (leukocyte acid phosphatase kit 387A, Sigma-Aldrich, Stockholm, Sweden) according to the manufacturer's instructions. TRAP positive cells with no less than 3 nuclei were counted manually as OCs using a light microscope. OCs derived from both Mϕ and iDC were grown in parallel on 96-well synthetic calcium phosphate coated plates (Corning, New York, USA). At the end of the culture, the supernatants were removed from the plate and erosion zones were visualized under a light microscope by removing the adherent OCs with chlorine bleach. OC activity was evaluated by measuring the resorption area in two random fields per well under low magnification using NIS elements software (Nikon Instruments Europe BV, Amsterdam, Netherlands).

IL-8 was neutralized in the cell supernatants using an anti-IL-8/CXCL8 neutralizing antibody (clone MAB208, R&D systems, UK). PAD activity was inhibited using a pan-PAD inhibitor Cl-amidine (Cayman chemical, Michigan, USA), either at the initiation of the OC cultures or three days before the end of culturing.

### IL-8 ELISA

During the priority year, IL-8 measurement was performed on Serum samples of Risk RA (n=44) and healthy individuals (n=44). Synovial fluid samples were collected from spondyloarthropathy (n=17), ACPA negative (n=13) and ACPA positive (n=17) RA patients and stored at -80°C until analysis. All samples were collected with informed consent from patient and patient diagnosis was defined by the American College of Rheumatology criteria/European League against Rheumatism. The sample collection and study was approved by the Karolinska Ethical Committee, Solna, Stockholm.

Human IL-8 ELISA was performed according to the manufacturer's instruction. Briefly, high protein binding ELISA plate was coated with primary antibody MT8H6 at concentration 2µg/ml in PBS and incubated overnight at 4-8°C. Plate was washed with PBS and blocked with PBS containing 0.05% tween 20 and 0.1% BSA for an hour. Samples or standards diluted in incubation buffer for the synovial fluid /Serum samples and incubated for 2 hours at room temperature. Plate was washed and incubated with secondary antibody MT8F19-biotin at 1 µg/ml for an hour. Streptavidin-HRP was incubated and developed with the substrate solution. Optical density was measured in an ELISA reader. In order to avoid the interference from heterophilic antibodies the synovial fluid samples were diluted at least 1:2 with Assay Buffer 3652-J2. As a specificity control, samples were run in parallel using ELISA plates coated with an irrelevant isotype control antibody, Ly128; mouse IgG1. All reagents were purchased from Mabtech AB, Stockholm Sweden.

### Synovial fibroblasts cultures and in vitro scratch assay

Synovial fibroblasts were isolated from the synovial tissue of RA patients by enzymatic digestion. The cells were growth at 37°C in 5% CO2 in Dulbecco's modified Eagle medium (DMEM, Sigma-Aldrich, Stockholm, Sweden) with 10% (v/v) heat-inactivated fatal cow serum (FCS, Sigma-Aldrich, Stockholm, Sweden), 100 U/ml penicillin, 100 µg/ml streptomycin and L-glutamine. The cells at passages 4-8 were used throughout this study. Twenty four-well plates were pre-coated with collagen (50 µg/ml), followed by 1 hour of blocking with 3% BSA (Sigma). A sufficient number of SFs were grown to 80-90% confluence and serum starved for 1-2 hours. The scratches were then made using a P-200 pipette tip. The floating cells were removed by washing with PBS. The cells were incubated with or without PAD inhibitors at the indicated concentration for 48 hours. Light microscope images were taken immediately at 0 and 5 hours after scratching. The images were analyzed using NIH ImageJ. The closure areas were normalized to medium control, and these values represent the migration index.

### Cytotoxicity assay

The LDH levels in the cell-free culture supernatants were measured using an LDH cytotoxicity assay kit (Roche Diagnostics Scandinavia AB, Bromma, Sweden) according to the manufacturer's instructions.

### Flow cytometry

For flow cytometric analysis, the cells were labeled using the anti CD14-fluorescein isothiocyanate (FITC) (Clone M5E2) and anti CD1a-phycoerythrin (PE) (clone HI149) and analyzed using a Gallios flow cytometer (Beckman Coulter, Stockholm, Sweden) and the Flow Jo software Version 9.2 (Ashland, OR, USA). The isotype controls were also included. All of the antibodies were purchased from BD Pharmingen (San Diego, CA, USA).

During the priority year, further studies of osteoclasts were performed: Cell at various stages of OC differentiation were stained for 30min at +4oC using 0.5X106 cells in 50 µl PBS. The following antibodies were used for CXCR1 and CXCR2 staining, all from Biolegend (San Diego, CA, USA):
PE-labelled anti-CXCR1 (clone 8F1/CXCR1);
PE-labelled mouse lgG2b isotype control (clone MPC-11);
APC-labelled anti-CXCR2 (clone 5E8/CXCR2); and
APC-labelled mouse IgG1 isotype control (MOPC-21)

The labeled cells were washed once in PBS and fixed using 1% paraformaldehyde. For dead cell exclusion the Live/dead fixable near-IR Dead Cell Stain Kit (Thermo Fisher) was used. Flow cytometry was performed using FACSVerse( Becton Dickinson, CA USA) and data were analysed with FlowJo v. 9 software (Tree Star Inc. Ashland, OR USA).

### PAD activity assay

Cell pellets were lysed with lysis buffer along with EDTA free-protease inhibitor by sonication for 5 minutes and centrifuged at 14000rpm for 15 minutes. Protein concentration was measured using DC protein assay (BIO-RAD, Stockholm, Sweden). PAD activity was measured using an antibody-based assay for PAD activity (ABAP) (Modi Quest Research, Netherlands), according to manufacturer instructions. Briefly, the cell lysates were added to arginine coated plate and the deiminated arginine was measured using MQR mouse anti-deiminated arginine antibody. Colorimetric changes were read at 450nm in a multiwell plate reader.

### Cytokine/chemokine analysis

The supernatants from the OC cultures were collected and stored at -20°C until analysis. Pro-inflammatory cytokine/chemokine production was determined using Cytometric bead array kits (CBA, BD Biosciences, San Diego, CA, USA) according to the manufacturer's instructions.

### Immunohistochemical analysis

Murinized monoclonal IgG2a ACPAs (D10, B02, C07) and control antibody (E02) were used to investigate the presence of citrullinated proteins during OC maturation. A rabbit polyclonal anti-PAD2 (Cosmo Bio, Tokyo, Japan) and a monoclonal mouse anti-PADI4 (Abcam, Cambridge, UK) antibody were used to investigate the cellular expression of PAD enzymes during OC maturation. Mϕ- and iDC-derived OCs were cultured in 8-well glass chamber slides.

During the priority year, further immunohistochemical analysis were performed. Mouse monoclonal antibody against CXCR1 (Abcam ab10400, Sweden) and CXCR2 (Abcam ab24963, Sweden) were used to investigate the presence of IL-8 receptors. The cellular expression of CXCR1 and CXCR2 was performed on different stages of development on MΦ derived OCs cultured in 8-well glass chamber slides.

Cells at different stages of differentiation were fixed with 2% (vol/vol) formaldehyde (Sigma-Aldrich, Stockholm, Sweden) at 4°C and stored at -70°C until use. Following blocking of endogenous peroxidase and avidin-biotin activity, the slides were incubated with primary monoclonal antibodies. HRP conjugated anti-mouse antibody was used as a secondary antibody and developed with 3,3-diaminobenzidene (DAB) for 7 minutes. The slides were counterstained with Mayer's hematoxylin, dehydrated and permanently mounted and viewed using a light microscope (Reichert Polyvar 2 type 302001, Leica).

### Mass spectrometry

Proteins were extracted from the cell pellets lysed in 8 M urea in 100 mM ammonium bicarbonate by sonication on ice. The protein concentrations were determined using the BCA method (BCA kit, Thermo Scientific, Bremen, Germany). Following reduction and alkylation, 10 µg of proteins was digested by trypsin at a ratio of 1:30 trypsin:protein in the presence of 1% ProteaseMAX (all reagents from Promega, Nacka, Sweden). The digestion was stopped with formic acid (FA). The digests were cleaned with Stage Tips (Thermo Scientific, Bremen, Germany), dried and resuspended in 0.1% FA prior to analysis. LC-MS/MS analyses were performed using an Easy-nLC chromatography system directly coupled on-line to a Q Exactive mass spectrometer (Thermo Scientific, Bremen, Germany).

The data was searched against a concatenated version of the complete proteome database using the Mascot search engine. The list of identified proteins was further filtered using 1% FDR. The proteomes were compared by performing a primary component analysis (PCA) of the normalized, log transformed protein areas using SIMCA 13.0.3 (Umetrics, Umeå, Sweden). Default settings were used with the exception of using Par scaling. Model performance was reported as cumulative correlation coefficients for the model (R2X[cum]) and predictive performance based on seven-fold cross validation calculations (Q2[cum]). By default, any proteins with missing values in 50% of the comparisons were removed.

### Animal experiments

Animal experiments were conducted using adult male Balb/c (Harlan) 15 weeks of age. Mice were housed in standard cages (3-5 per cage) in a climate controlled environment maintaining a 12-hour light/dark cycle with access to food and water ad libitum. All experiments were approved by the local ethics committee for animal experiments in Sweden. Mice were injected (i.v.) with either saline or mAb ACPA (2 mg, equal mixture of D10 and B02) diluted in 100 µl saline. Starting day 6, the CXCR2 antagonist reparixin (L-lysin salt, HY-15252, MedChem Express) was injected subcutaneously (s.c. in 100 µl saline) twice daily (30 mg/kg/day) for 6 days. At the end of the study, mice were anesthetized using 4% isoflurane, decapitated and left hind leg removed and post-fixed in 4% PFA until further analysis.

Bone structure was analyzed using a SkyScan 1176 micro-CT (Bruker) with a voxel size of 9 µm. The scanning was conducted at 50kV/480µA with a 0.2mm aluminum filter. The exposure time was 900 ms. The x-ray projections were obtained at 0.4° intervals with a scanning angular rotation of 180°. The projection images were reconstructed into 3-dimensional images using NRecon software (version 1.6.9.8; Bruker) and analyzed using CTVox software (version 2.7.0; Bruker). Trabecular bone in tibia located 644µm from the proximal growth plate and extending 100,5µm was analysed regarding BMD and 3D analysis and a volume of cortical bone in tibia mesasuring 617µm in length, located in the distal tibia was measured for TMD, using CTAnalyzer software (version 1.14.4.1; Bruker). The 3D structures of each joint were blindly assessed by two observers (T.J. and M.M.).

### Gene expression analysis

Another study performed during the priority year relates to gene expression analysis of osteoclasts during different stages of differentiation.

RNA isolation was performed at various stages of OC differentiation using the RNeasy Plus Mini Kit of Qiagen, following the manufacturer's instruction. RNA concentrations were measured using Nanodrop 1000 (NanoDrop, Wilmington, DE, USA) and cDNA was synthetized using the High Capacity Reverse Transcription Kit of Applied Biosystems (Thermo Fisher Scientific, Waltham, MA USA). For Real-Time PCR we used the following gene expression assays of Applied Biosystems: Hs00174103_m1 (IL8), Hs01921207_s1 (CXCR1), Hs01891184_s1 (CXCR2) in addition to AmpliTaq DNA Polymerase with buffer I., dNTP Set 100mM Solutions and ROX reference dye (all from Thermo Fisher Scientific). PCR conditions were set as recommended for Applied Biosystem gene expression assays and the assays were run on QuantStudion 7 Flex (Applied Biosystems). Expression levels were normalized to cyclophilin expressions, quantified using the following primers all synthetized by Integrated DNA Technologies (Leuven, Belgium):
5'-ACGGCGAGCCCTTGG-3'
5'-TTTCTGCTGTCTTTGGGACCT-3',
5'-/56-FAM/CGCGTCTCCTTTGAGCTGTTTGCA/3BHQ_1/-3'

### Statistical analysis

Mean differences between groups were compared using either one-way or two-way ANOVA followed by Tukey's post-hoc test, using GraphPad Prism 6 software. P values less than 0.05 was considered significant.

### Results

### 1. Polyclonal ACPAs derived from both peripheral blood (PB) and synovial fluid (SF) promote osteoclastogenesis

To test the effects of ACPAs on osteoclastogenesis, CD14-positive monocytes were purified from the PB of healthy individuals and RA patients. Monocytes were differentiated first to MΦ in the presence of M-CSF and then to mature OC in the presence of RANKL and M-CSF. ACPAs were obtained via affinity purification from either PB or synovial fluid (SF) of ACPA-positive RA patients using affinity columns conjugated with CCP-2 peptides (Ossipova, et al., 2014). Both ACPA pools reacted with a large number of different citrullinated peptides from different putative autoantigens as detected by a multiplex chip-based assay (Hansson et al., 2012). (See also Figure 1A).

PB- as well as SF-derived ACPA IgG pools but not control IgGs (flow-through fractions of the CCP-2 affinity columns, i.e. CCP-2 non-reactive IgGs) were effective in inducing osteoclastogenesis from PB-derived MΦ of healthy individuals (a mean fold increase in the osteoclast numbers of 1.9±0.3 for PB-derived ACPA and 1.9±0.2 for SF-derived ACPA compared to those of controls, p< 0.05, Figure 1B). Similar results were obtained when OCs were obtained from PB-derived macrophages of ACPA-positive RA patients (data not shown).

### 2. Monoclonal ACPAs derived from single synovial B cells have variable effects on osteoclastogenesis

Because both the PB and SF ACPA pools contained a wide spectrum of human antibodies with a distinct fine specificity for multiple epitopes of cit-proteins, the present inventors wanted to investigate whether ACPAs with different characteristics might differ in their osteoclastogenic effect. To this end, the present inventors utilized single B/plasma cell-derived ACPA monoclonal antibodies and tested their effects on osteoclastogenesis and bone resorption. The present inventors selected 4 monoclonal antibodies that react with cit, but not unmodified, forms of fibrinogen (fib) 36-52, enolase 5-21 (CEP1) and vimentin (vim) 60-75 peptides, and a control antibody reacting with the tetanus toxoid antigen aa1300-1314 but with none of the cit-peptides.

The control E02 antibody as well as two ACPAs monoclonals (B09 reacting with only cit-fib 36-52 and C07 reacting with CEP1 and reacting more weakly with cit-vim 60-75) antibodies showed no effect on either osteoclastogenesis or bone destruction. In contrast, two other ACPAs monoclonals (D10 and B02, both showing reactivity with cit-vim 60-75 and CEP1) enhanced both OCs formation (a fold increase of 2.0±0.1 for both B02 and D10 compared to the control E02 antibody) and bone resorption area (a fold increase of 2.0±0.2 for B02 and 1.4±0.1 for D10 compared to the control E02 antibody).

To further investigate the relevance of antibody specificity in mediating osteoclastogenesis, monomeric Fab fragments of the two active antibodies (D10 and B02) and the E02 control antibody were generated using a similar cloning technology as that used for the full antibodies. The Fab fragments of both D10 and B02 but not E02 antibodies were able to promote osteoclastogenesis (a fold increase of 1.8±0.3 for B02 and 1.8±0.2 for D10) and bone destruction (a fold increase of 2.1±0.2 for B02 and 2.1±0.1 for D10).

### 3. Role of citrullination and PAD enzymes in OC differentiation with and without ACPA stimulation

The differential osteoclastogenic effect of polyclonal as well as monoclonal ACPAs but not of the control anti tetanus toxin antibody suggests that citrullination might be an important event in developing OCs. To examine this possibility, the present inventors first investigated citrullination patterns during OC development using murinized monoclonal ACPAs, i.e., where the human Fc part was exchanged for a murine IgG2a Fc (Amara et al., 2013) in order to enable immunostainings of human cells. Both MΦ precursors and MΦ-derived mature OCs stained positively for the B02 and D10 monoclonal ACPAs but did not stain for the C07 ACPA antibody or the E02 control antibody. No staining with either of the antibodies was detected in the CD14-positive cells from which MΦ were originally developed (Figure 2A). The staining intensity increased in the more mature osteoclasts and markedly diminished after OCs treatment with the PAD inhibitor Cl-amidine (Figure 2B).

Subsequently, the inventors investigated the presence of PAD2 and PAD4 in OCs in different differentiation stages using monoclonal antibodies specific for these enzymes. Antibodies against PAD2 and PAD4 showed faint staining in CD14 monocytes with increased staining intensity in both MΦ-precursors and more mature OCs. Using an antibody-based ELISA assay as described in Zendman *et al.,* 2007, significant PAD activity was detected during all stages of OC development, with lower levels in cell lysates of mature OCs than of MΦ-precursor, suggesting a role for these enzymes during OC maturation and development. This result was confirmed by the dose-dependent inhibition of OC differentiation using Cl-amidine, a PAD2/PAD4 inhibitor (PADi) in the presence of RANKL and M-CSF, without inducing cell death, as evaluated by LDH release in the supernatants. In contrast, no changes in cell phenotype (fibroblast migration) or survival (LDH assay) were observed when RA-derived synovial fibroblasts (used as a control cell population) were incubated with PADi at similar doses, indicating a cell-type specific dependency on PAD enzymes for the normal differentiation and proliferation of OCs.

ACPAs were not able to promote OCs activation when PADi was added from the beginning of the cultures. Interestingly, doses as low as 0.2 µM PADi were no longer able to affect the unstimulated differentiation of OCs, but were still able to inhibit ACPA-mediated osteoclastogenesis. Time kinetic experiments, using OCs precursor from the same donor, showed that early (at the initiation of the OC culture) and late (three days before ending the OC cultures, figure 3E) incubation with PADi had different effects. Early inhibition affects both unstimulated and ACPA-mediate osteoclastogenesis, while late inhibition affects only ACPA-mediated osteoclastogenesis, even at doses as high as 20 µM.

### 4. IL-8 is an essential mediator of A CPA-driven osteoclastogenesis

To investigate potential mediators responsible for ACPAs effect, we analyzed a set of common cytokines known to regulate osteoclastogenesis in cell culture supernatants. IL-6, IL-1, IL-10 and TNF-alpha were detected at low basal levels and showed no consistent changes during OC development with or without ACPA treatment (Results not shown). In contrast, high levels of IL-8 were detected in MΦ-derived OC cultures at early times during their maturation (2426±29 pg/ml at day 4) and further increased with time (5532±98 pg/ml at day 6 and 9858±387 pg/ml at day 12). ACPA, but not control IgGs further increased IL-8 release in the culture supernatants over time (Figure 4A).

The present inventors tested whether IL-8 is involved in ACPA-driven osteoclastogenesis. As shown in Figure 4B, the blockade of extracellular IL-8 with a neutralizing and IL-8-specific antibody in the presence of M-CSF and RANKL dose dependently blocked the differentiation of immature osteoclasts into mature osteoclasts (Figure 4C) and was also able to block the effects of ACPA at doses as low as 1 µg/ml (Figure 4D). Blocking of ACPAs effects was observed when the neutralizing anti-IL-8 antibody was added either at the beginning (first 3 days) or at the end of the cultures (the last 3 days). No such effects were observed when TNF-alpha was blocked with adalimumab even at higher concentrations (10 µg/ml, Figure 4E).

### 5. ACPAs effects on osteoclastogenesis are independent of the OC-precursor cell phenotypes

As immature dendritic cells (iDC) develop into OCs more efficiently than monocytes and iDC but not MΦ transdifferentiate into OCs in the presence of cell free RA SF 25, the present inventors further investigated whether ACPAs' effects are dependent on the cell phenotype of the OC precursors. To this end, non-adherent iDCs were generated by from CD14 positive monocytes of healthy individuals and ACPA+ RA patients, in the presence of IL-4 and GM-CSF and were subsequently developed into OCs in the presence of RANKL and M-CSF. Proteomic profiling during different stages of differentiation showed that the profiles of maturing OCs with iDC origin converged over time with those of OCs with MΦ origin though through distinct maturation pathways.

Similar to MΦ precursors, ACPA IgGs were able to promote osteoclastogenesis from iDC precursors with a significant increase in both osteoclast numbers (a fold increase of 2.3±0.9, p<0.05) and bone resorption area (a fold increase of 2.6±0.9, p<0.05) compared to control IgGs. Both iDC precursors and iDC-derived mature OCs stained positively for the B02 antibody but not for the C07 ACPA antibody or the E02 control antibody, suggesting again that citrullination is important for OC differentiation and maturation.

Similar to MΦ-derived OC, PAD2 and PAD4 showed faint staining in CD14 monocytes with increased staining intensity in both iDC-precursors and more mature OCs. The importance of citrullination and PAD enzymes for iDC transdifferentiation was confirmed by a dose-dependent inhibition of OC differentiation using Cl-amidine, a PAD2/PAD4 inhibitor (PADi), in the presence of RANKL and M-CSF, similar to our observation for MΦ-derived osteoclastogenesis.

Similar to MΦ-derived OC cultures, IL-8 was the main cytokine detected in the culture supernatants of iDC-derived cultures although at lower basal levels compared to MΦ-derived OC (425±71 pg/ml at day 4). Additionally, a significant increase in the IL-8 levels was also observed in these cultures following ACPA treatment at all time points tested, with a maximum increase during the early time points (1555±158 pg/ml at day 4).

### 6. In vivo ACPA-induced systemic bone loss is reversed by an IL-8 antagonist

The inventors tested whether ACPAs can induce bone loss in vivo using micro-CT evaluation of the tibia in control mice (Figure 5A) and mice injected with murinized monoclonal ACPAs alone (Figure 5B) or together with a CXCR1/2 antagonist (reparixin) blocking the murine IL-8 homologues (Figure 5C). ACPA i.v. injection significantly decreased the trabecular bone mineral density (BMD, Figure 5D), the trabecular number (Figure 5E) and the bone volume fraction (bone volume/tissue volume, Figure 5F), while not affecting the cortical tissue mineral density (TMD, figure 5G). Changes were reversed by s.c. administration of reparixin (figure 5D-F). Histological examination of joint tissues revealed minimal signs of synovitis in only one of the 9 ACPA-treated mice, whereas no changes were seen in joint tissues from the other 16 animals.

### 7. Experiments performed during the priority year

### A) BB-CI-amidine has dose dependent effect in vitro

During the priority year, the inventors tested the commercially available PAD inhibitor BB-CI-amidine (N-[(1S)-1-(1H-benzimidazol-2-yl)-4-[(2-chloro-1-iminoethyl)amino]butyl]-[1,1'-biphenyl]-4-carboxamide) in the ACPA non-stimulated OC *in vitro* assay described herein, including an evaluation using both the TRAP assay and a bone erosion assay.

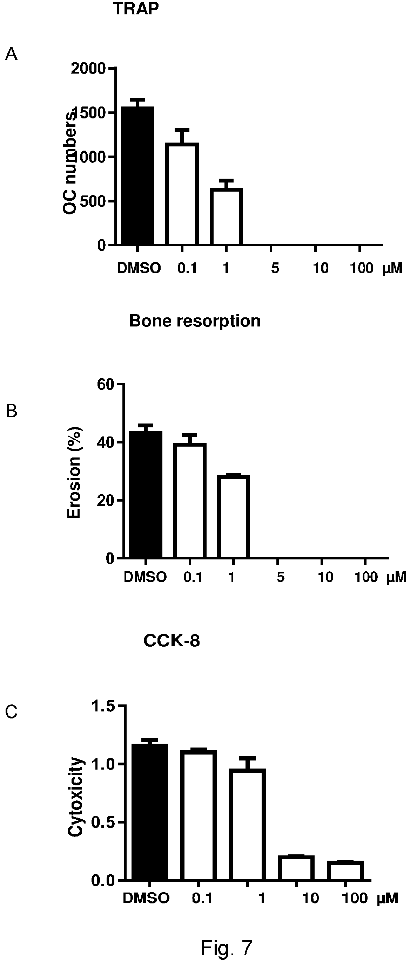

The results are shown in Fig. 7, where the results with regard to osteoclast numbers and erosion (%) clearly show a dose dependent effect at concentrations 0.1 and 1 µM, but also indicate a cytotoxic effect at 10 µM as evaluated using CCK-8.

### B) Proprietary PAD inhibitors exhibit dose dependent effect in vitro

The inventors also evaluated two proprietary selective PAD2 inhibitors, and four selective PAD4 inhibitors in the same ACPA non-stimulated OC in vitro assay described herein, including an evaluation using both the TRAP assay and a bone erosion assay. The results indicate a dose dependent effect on osteoclast number and erosion (%) but no or only limited cytotoxicity. (Results not shown.)

One selective PAD2 inhibitor and one selective PAD4 inhibitor were evaluated in ACPA stimulated OC assays *in vitro.* For the PAD2 inhibitor, a dose dependent effect was seen, and for both the PAD2 and PAD4 inhibitors, it was shown that the effect of ACPA stimulation could be neutralized. (Results not shown.)

### C) Comparison of reparixin and reparixin L-lysine

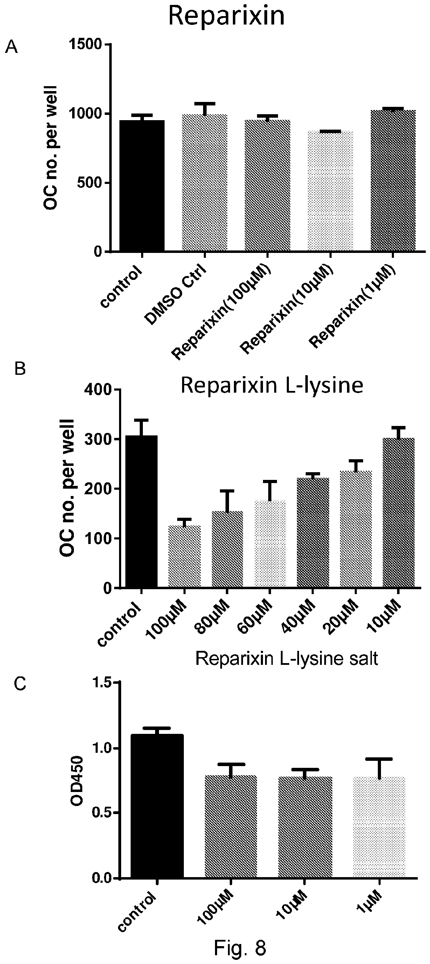

The above two forms of reparixin were tested in the ACPA non-stimulated OC in vitro assay described herein. In this experiment, reparixin did not show any effect, whereas reparixin L-lysine exhibited a dose dependent inhibition in the tested dose range (10 - 100 µM). The results are shown in Fig. 8.

### D) Testing two experimental IL-8 inhibitors

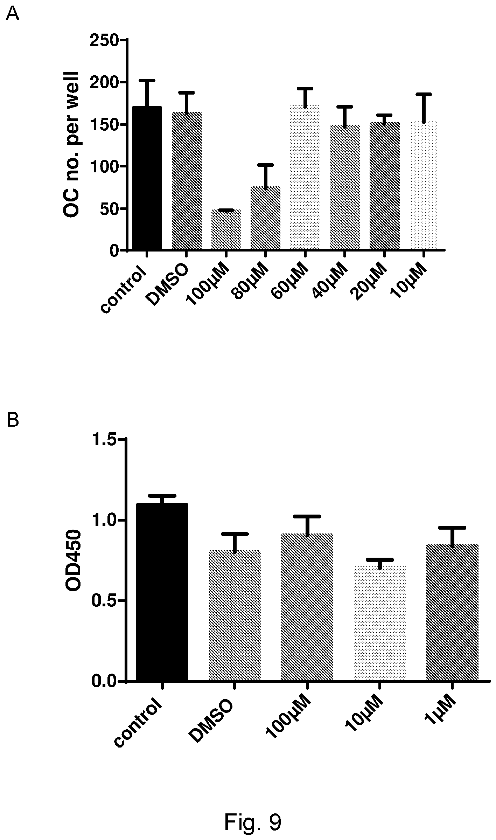

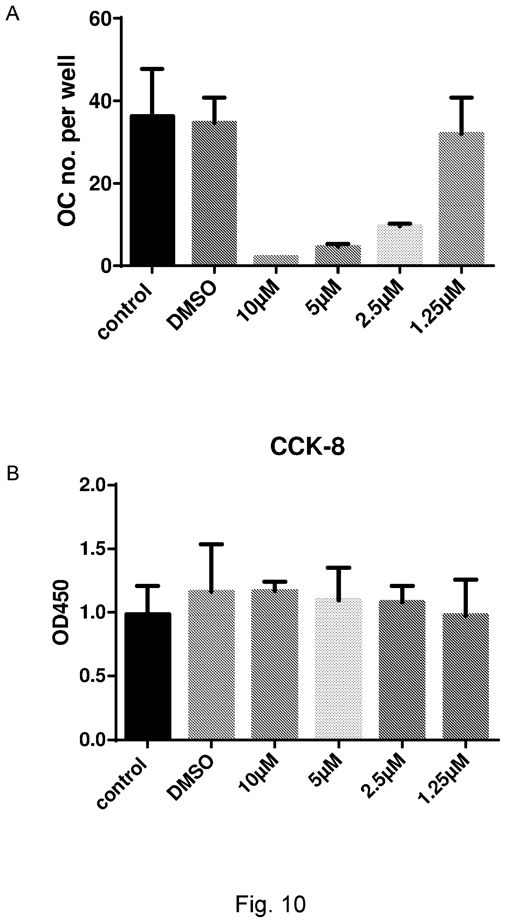

The commercially available IL-8 inhibitors SCH 527123 and SB 332235 were tested in the ACPA non-stimulated OC in vitro assay described herein. As shown in Fig. 9 and 10, SB 527123 showed some inhibition at high doses (80 and 100 µM) whereas SB 332235 exhibited a clear dose dependent effect over the concentration interval tested (1.25 - 10 µM)

### E) IL-8 in serum and synovial fluid

Analysis of patient samples showed interesting results. Synovial fluid of ACPA-positive RA patients appears to contain higher levels of IL-8 as compared to ACPA-negative RA patients and patients with other inflammatory joint diseases than RA such spondyloarthritis patients. Synovial levels of IL-8 were shown to be elevated in RA patients with ACPA positive RA as compared to RA patients without ACPA and patients with other inflammatory joint diseases such as spondylarthorpaties. IL-8 concentrations ranging from 0 to 5000 pg/ml were measured in patients with spondylarthorpaties, with an average just below 5000 pg/ml. In ACPA negative RA patients, IL-8 concentrations up to 50 000 pg/ml were detected, with an average just above 5000 pg/ml. In ACPA positive RA patients, the IL-8 concentrations averaged around 10 000 pg/ml.

Serum IL-8 levels appear to be significantly higher in the serum of ACPA-positive patients with arthralgia, as compared to ACPA-negative healthy individuals. Serum IL-8 levels were shown to be elevated in individuals at risk for developing arthritis as compared to matched controls. In ACPA positive patients not yet developing RA, IL-8 concentrations up to 40 pg/ml were detected, and the average IL-8 concentration was found to be about 5 pg/ml. In ACPA positive patients who had developed RA, slightly elevated concentrations were detected. In healthy controls, the spread was larger, but the average IL-8 concentration was significantly lower than in the two previous groups.

### F) Immunohistochemical analysis of intracellular and extracellular IL-8

Immunohistochemistry staining showed high amounts of intracellular IL-8 in developing OCs in culture, as observed at 0, 24, 72, 96 and 168 hours. Similarly, immunohistochemistry staining indicated presence of extracellular IL-8 in developing OCs, as observed on day 3, 6 and 13

### G) Immunohistochemical analysis of intracellular and extracellular

Immunohistochemistry stainings confirmed the cell surface presence of CXCR1 and to a lesser extent of CXCR2. Immunohistochemistry stainings on permeabilized cells show high amounts of both CXCR1 and CXCR2 during all stages of OC maturation.

### H) Flow cytometry

Flow cytometry results showed that CXCR1 and to a lesser extent CXCR2 are detected on the cell surface of developing OCs.

### I) rtPCR expression of CXCR1/2 and IL-8

Real time PCR analysis showed presence of CXCR1 and CXCR2 genes in developing OCs in culture, Similarly, rtPCR showed high IL-8 gene expression at the beginning of OC development.

### Discussion

A major new and surprising finding in this disclosure is that ACPA-induced OC maturation and bone resorption leads to the preferential production of IL-8 but not several other proinflammatory cytokines and that IL-8 is also necessary for further maturation and bone resorption activities of OCs, thereby serving as an autocrine regulator after ACPA stimulation. Another novel finding is that PAD enzymes appear to be necessary for osteoclast activation and bone erosion not only after stimulation with ACPAs but also in the absence of such stimulus. A third finding extending from previous observations is that some but not all monoclonal antibodies generated from B cells/plasma cells from inflamed RA joints and also Fab fragments of these antibodies stimulate osteoclast activation and bone erosion.

The present inventors have thus demonstrated that ACPAs purified on CCP-2-linked affinity columns promote osteoclastogenesis, irrespective of whether antibodies are purified from PB or from SF. This finding is in line with the report of Harre et al. 2012, who used serum-derived Abs purified using affinity columns with MCV 20. The lack of OCs promoting effect of the flow-through IgG fractions, of not only PB but also SF, shows that indeed only antibodies specifically recognizing citrullinated epitopes (but not other antibodies from rheumatoid joint) enhance OC activation. The present inventors however demonstrated that antibody fine specificities matter, since different monoclonal antibodies had different effects on osteoclastogenesis.

The present inventors also demonstrated that cit-vim (not only MCV but also cit-vim 60-75) is an important ACPA target during OC differentiation. Additionally, the Fab fragments of the monoclonals promoted OC activation, suggesting that ACPAs effect is at least partly Fc-receptor-independent.

The studies on OC differentiation and maturation allowed the inventors to demonstrate that PAD2 as well as PAD4 were prominently expressed in all stages of osteoclast maturation. This pattern of PAD expression is compatible with the presence of cit-epitopes, as detected by our monoclonal ACPAs in all stages of OC differentiation. The present inventors provide further evidence that ACPAs effects are due to the recognition of citrullinated epitopes generated during osteoclast differentiation, since PAD inhibition completely eliminates the OC-activating effects of these antibodies. Notably, however, the inhibition of PADs also prevented normal osteoclast differentiation in the absence of ACPAs when used in early stages of OC development.

This observation suggests that one or several PADs and thus citrullination may have unique functions during OC differentiation, including functions that are not present in other cells (as shown here for synovial fibroblasts). Such a tentative unique feature of OC differentiation can be hypothesized to explain the presence of cit-proteins within and on the cell surface of OCs during their normal differentiation, in contrast to most other cells that express cit-proteins mainly in the context of inflammation. Such a dependency on PADs and citrullination for normal OC differentiation might therefore also explain how OCs can be preferentially targeted by ACPAs in a non-inflammatory context. Interestingly, the osteoclastogenesis dependency on both citrullination and PAD was observed independent of the cell phenotype of the OC-precursors (either MΦ or iDC). Common MΦ and DC precursors able to transdifferentiate into OCs are present in the bone marrow of healthy individuals and enriched in the bone marrow of patients with inflammatory bone erosions (Chiu et al., 2012).

The detailed molecular mechanisms responsible for the ACPA-induced osteoclast activation are so far relatively unknown. The present demonstration that IL-8 is by far the dominating cytokine/chemokine (out of the standard set measured here) released from ACPA-stimulated OCs of both MΦ and iDC precursors and that IL-8 also appears to function in an autocrine fashion provides a new and interesting insight.

IL-8 production by OCs has been described before (Rothe *et al.,* 1998) and was recently proposed to have an autocrine effect on osteoclastogenesis (Kopesky *et al*.,2014) but not in the context of ACPA stimulation. The central role of IL-8 in ACPA-induced OC activation in a context where a low production of TNF, IL-1 or IL-6 is observed is thus in line with the clinical as well as experimental observation that OC activation and bone erosion may occur due to ACPA stimulation also in the absence of the more conventional pro-inflammatory cytokines (Kleyer et al., 2013; Harre et al., 2012).

In conclusion, the observations of the effects of ACPAs on OCs support a novel, testable hypothesis for how extra-articular generated ACPAs might specifically target the joints and contribute to RA-specific joint lesions (Figure 6). Thus, the cell-specific requirement of PAD for normal OC differentiation and the calcium-rich bone marrow environment lead to increase citrullination (despite no inflammation) and allow an initial specific targeting of bone marrow OC precursors by circulating ACPAs. This leads to increased amounts of IL-8 that further stimulates OCs through an autocrine loop.

Notably, one recent study of the same inventors has shown that ACPAs are able to increase IL-8 joint production and to induce pain-like behaviors when injected in mice (Camilla Svensson et al, co-pending international application claiming priority from US Provisional Patent Application Serial Number 62/188,499). In a second step, communication between bone marrow and synovium through bony canaliculi present at the cartilage-bone junction (Marinova-Mutafchieva et al., 2002) will allow IL-8 to migrate to the joint. In the joint, IL-8 (also called neutrophilin) will initiate chemoattraction and migration of inflammatory cells, in particular neutrophils that initiate early stages of synovitis. This scenario together with the recent finding that ACPAs promote release of neutrophil extracellular traps (NETs) from neutrophils and augment inflammatory responses in synovial fibroblasts (Khandpur *et al.,* 2013) suggests a convergence of two different ACPA-dependent events at the interphase between the bone surface and synovium, i.e., OC and neutrophil activation synergizing to promote bone erosion and local inflammation. Such a scenario might help answer the long-standing question of why and how ACPAs may specifically contribute to joint inflammation and not inflammation elsewhere and why initial lesions often occur at the site where bone and synovium meet.

### References

Amara, K., et al. Monoclonal IgG antibodies generated from joint-derived B cells of RA patients have a strong bias toward citrullinated autoantigen recognition. J Exp Med 210, 445-455 (2013).
Chiu, Y.H., et al. Regulation of human osteoclast development by dendritic cell-specific transmembrane protein (DC-STAMP). J Bone Miner Res 27, 79-92 (2012).
Hansson, M., et al. Validation of a multiplex chip-based assay for the detection of autoantibodies against citrullinated peptides. Arthritis Res Ther 14, R201 (2012).
Harre, U., et al. Induction of osteoclastogenesis and bone loss by human autoantibodies against citrullinated vimentin. J Clin Invest 122, 1791-1802 (2012).
Khandpur, R., et al. NETs are a source of citrullinated autoantigens and stimulate inflammatory responses in rheumatoid arthritis. Sci Transl Med 5, 178ra140 (2013).
Kleyer, A., et al. Bone loss before the clinical onset of rheumatoid arthritis in subjects with anticitrullinated protein antibodies. Ann Rheum Dis (2013).
Kopesky, P., et al. Autocrine signaling is a key regulatory element during osteoclastogenesis. Biology open 3, 767-776 (2014).
Makrygiannakis, D., et al. Citrullination is an inflammation-dependent process. Ann Rheum Dis 65, 1219-1222 (2006).
Marinova-Mutafchieva, L., Williams, R.O., Funa, K., Maini, R.N. & Zvaifler, N.J. Inflammation is preceded by tumor necrosis factor-dependent infiltration of mesenchymal cells in experimental arthritis. Arthritis Rheum 46, 507-513 (2002).
Mechin, M.C., et al. The peptidylarginine deiminases expressed in human epidermis differ in their substrate specificities and subcellular locations. Cellular and molecular life sciences : CMLS 62, 1984-1995 (2005).
Molenaar, E.T., et al. Progression of radiologic damage in patients with rheumatoid arthritis in clinical remission. Arthritis Rheum 50, 36-42 (2004).
Nasi, A., et al. Dendritic cell reprogramming by endogenously produced lactic acid. The Journal of Immunology 191, 3090-3099 (2013)
Ossipova, E., et al. Affinity purified anti-citrullinated protein/peptide antibodies target antigens expressed in the rheumatoid joint. Arthritis research & therapy 16, R167 (2014).
Rantapää-Dahlqvist S, de Jong BA, Berglin E, Hallmans G, Wadell G, Stenlund H, Sundin U, van Venrooij WJ, Antibodies against cyclic citrullinated peptide and IgA rheumatoid factor predict the development of rheumatoid arthritis, Arthritis Rheum. 2003 Oct; 48(10):2741-9
Rothe, L., et al. Human osteoclasts and osteoclast-like cells synthesize and release high basal and inflammatory stimulated levels of the potent chemokine interleukin-8. Endocrinology 139, 4353-4363 (1998).
Takahara, H., Okamoto, H. & Sugawara, K. Calcium-Dependent Properties of Peptidylarginine Deiminase from Rabbit Skeletal-Muscle. Agricultural and Biological Chemistry 50, 2899-2904 (1986).
Teitelbaum, S.L. Bone resorption by osteoclasts. Science 289, 1504-1508 (2000).
Vossenaar, E.R., Zendman, A.J.W., van Venrooij, W.J. & Pruijn, G.J.M. PAD, a growing family of citrullinating enzymes: genes, features and involvement in disease. BioEssays : news and reviews in molecular, cellular and developmental biology 25, 1106-1118 (2003).
Vossenaar, E.R., et al. The presence of citrullinated proteins is not specific for rheumatoid synovial tissue. Arthritis Rheum 50, 3485-3494 (2004).
Zendman, A.J., et al. ABAP: antibody-based assay for peptidylarginine deiminase activity. Analytical biochemistry 369, 232-240 (2007).

## Claims

1. A compound capable of inhibiting the activity of peptidylarginine deiminase (PAD) enzymes (a PAD-inhibitor) for use in the alleviation of pain associated with an elevated activation of osteoclasts in a subject wherein said elevated activation of osteoclasts is associated with the presence of anti-citrullinated protein antibodies (ACPA) in the subject, and wherein said anti-citrullinated protein antibodies (ACPA) are detectable in a sample taken from a subject, but wherein the subject does not manifest clinical signs of rheumatoid arthritis.

2. The PAD-inhibitor for use according to claim 1, wherein said PAD inhibitor is an amidine compound.

3. The PAD-inhibitor for use according to claim 1, wherein said PAD inhibitor is an amidine compound chosen from:
F-amidine (N-[(1S)-1-(aminocarbonyl)-4-[(2-fluoro-1-iminoethyl)amino]butyl]-2,2,2-trifluoroacetate-benzamide);
Cl-amidine (N-α-benzoyl-N5-(2-chloro-1-iminoethyl)-L-Orn amide);
BB-CI-amidine (N-[(1S)-1-(1H-benzimidazol-2-yl)-4-[(2-chloro-1-iminoethyl)amino]butyl]-[1,1'-biphenyl]-4-carboxamide);
TDFA (Thr-Asp-F-amidine);
BTT-Cl-amidine (biphenyl tetrazole tert-butyl Cl-amidine);
o-F-amidine (N-α-(2-carboxyl)benzoyl-N(5)-(2-fluoro-1-iminoethyl)-l-ornithine amide); and
o-CI-amidine (N-α-(2-carboxyl)benzoyl-N(5)-(2-chloro-1-iminoethyl)-l-ornithine amide).

4. The PAD-inhibitor for use according to claim 1, wherein said compound is streptonigrin (SID 11532976).

5. A diagnostic kit for use in identifying individuals that would benefit from a method of alleviating pain in a subject wherein said pain is associated with an elevated activation of osteoclasts in said subject and wherein said elevated activation of osteoclasts is associated with the presence of autoantibodies in said subject, wherein said method and/or kit comprises one or more of the following:
- an assay for determining the level of osteoclast activation,
- an assay for determining the presence and identity of autoantibodies, including presence of antibodies to citrullinated antigens, and
- a questionnaire for quantitatively and optionally qualitatively assessing pain, and in particular joint pain (arthralgia), and optionally also
- an assay, the means for, or a step of qualitatively or quantitatively assessing bone density.

6. The kit for use according to claim 5, further comprising an assay for qualitatively and/or quantitatively assessing the presence of rheumatoid factors in a sample taken from said individual.

## Patentansprüche

1. Verbindung, fähig zum Hemmen der Aktivität von Peptidylarginindeiminase(PAD)-Enzymen (ein PAD-Hemmer) zur Verwendung bei der Linderung von Schmerz, der mit einer erhöhten Aktivierung von Osteoklasten in einem Subjekt verbunden ist, wobei die erhöhte Aktivierung von Osteoklasten mit dem Vorhandensein von Anti-citrulliniertes-Protein-Antikörpern (ACPA) in dem Subjekt verbunden ist, und wobei die Anti-citrulliniertes-Protein-Antikörper (ACPA) in einer von einem Subjekt genommenen Probe nachweisbar sind, wobei das Subjekt aber keine klinischen Zeichen von rheumatoider Arthritis zeigt.

2. PAD-Hemmer zur Verwendung gemäß Anspruch 1, wobei der PAD-Hemmer eine Amidinverbindung ist.

3. PAD-Hemmer zur Verwendung gemäß Anspruch 1, wobei der PAD-Hemmer eine Amidinverbindung ausgewählt aus:
F-Amidin (N-[(1S)-1-(Aminocarbonyl)-4-[(2-fluor-1-iminoethyl)amino]butyl]-2,2,2-trifluroacetatbenzamid) ;
Cl-Amidin (N-α-Benzoyl-N5-(2-chlor-1-iminoethyl)-L-Orn-amid) ;
BB-Cl-Amidin (N-[(1S)-1-(1H-Benzimidazol-2-yl)-4-[(2-chlor-1-iminoethyl)amino]butyl]-[1,1'-biphenyl]-4-carboxamid);
TDFA (Thr-Asp-F-Amidin);
BTT-Cl-Amidin (Biphenyltetrazol-tert-butyl-Cl-amidin);
o-F-Amidin (N-α-(2-Carboxy)benzoyl-N(5)-(2-fluor-1-iminoethyl)-1-ornithinamid); und
o-Cl-Amidin (N-α-(2-Carboxy)benzoyl-N(5)-(2-chlor-1-iminoethyl)-l-ornithinamid)
ist.

4. PAD-Hemmer zur Verwendung gemäß Anspruch 1, wobei die Verbindung Streptonigrin (SID 11532976) ist.

5. Diagnostisches Kit zur Verwendung zum Identifizieren von Individuen, die von einem Verfahren zum Lindern von Schmerz in einem Subjekt Nutzen ziehen würden, wobei der Schmerz mit einer erhöhten Aktivierung von Osteoklasten in dem Subjekt verbunden ist und wobei die erhöhte Aktivierung von Osteoklasten mit dem Vorhandensein von Autoantikörpern in dem Subjekt verbunden ist, wobei das Verfahren und/oder Kit eines oder mehrere von Folgendem umfasst:
- ein Assay zum Bestimmen des Grads an Osteoklastenaktivierung,
- ein Assay zum Bestimmen des Vorhandenseins und der Identität von Autoantikörpern, einschließlich des Vorhandenseins von Antikörpern für citrullinierte Antigene, und
- einen Fragebogen für die quantitative und gegebenenfalls qualitative Beurteilung von Schmerz, insbesondere Gelenkschmerz (Arthralgie), und gegebenenfalls auch
- ein Assay, die Mittel oder einen Schritt zur qualitativen oder quantitativen Bestimmung von Knochendichte.

6. Kit zur Verwendung gemäß Anspruch 5, ferner umfassend ein Assay zur qualitativen und/oder quantitativen Bestimmung des Vorhandenseins von rheumatoiden Faktoren in einer von dem Individuum genommenen Probe.

## Revendications

1. Composé capable d'inhiber l'activité d'enzymes peptidylarginine déiminase (PAD) (inhibiteur de PAD) pour une utilisation dans le soulagement d'une douleur associée à une activation élevée d'ostéoclastes chez un sujet, ladite activation élevée d'ostéoclastes étant associée à la présence d'anticorps anti-protéines citrullinées (ACPA) chez le sujet, et lesdits anticorps anti-protéines citrullinées (ACPA) étant détectables dans un échantillon prélevé d'un sujet, mais le sujet ne manifestant pas de signes cliniques d'arthrite rhumatoïde.

2. Inhibiteur de PAD pour une utilisation selon la revendication 1, ledit inhibiteur de PAD étant un composé de type amidine.

3. Inhibiteur de PAD pour une utilisation selon la revendication 1, ledit inhibiteur de PAD étant un composé de type amidine choisi parmi :
F-amidine (N-[(1S)-1-(aminocarbonyl)-4-[(2-fluoro-1-iminoéthyl)amino]butyl]-2,2,2-trifluoroacétate-benzamide) ;
Cl-amidine (N-α-benzoyl-N5-(2-chloro-1-iminoéthyl)-L-Orn amide) ;
BB-Cl-amidine (N-[(1S)-1-(1H-benzimidazol-2-yl)-4-[(2-chloro-1-iminoéthyl)amino]butyl]-[1,1'-biphényl]-4-carboxamide) ;
TDFA (Thr-Asp-F-amidine) ;
BTT-Cl-amidine (biphényl-tétrazole tert-butyl-Cl-amidine ) ;
o-F-amidine (N-α-(2-carboxyl)benzoyl-N(5)-(2-fluoro-1-iminoéthyl)-I-ornithine amide) ; et
o-Cl-amidine (N-α-(2-carboxyl)benzoyl-N(5)-(2-chloro-1-iminoéthyl)-I-ornithine amide).

4. Inhibiteur de PAD pour une utilisation selon la revendication 1, ledit composé étant la streptonigrine (SID 11532976).

5. Kit de diagnostic pour une utilisation dans l'identification d'individus qui pourraient bénéficier d'un procédé de soulagement d'une douleur chez un sujet, ladite douleur étant associée à une activation élevée d'ostéoclastes chez ledit sujet et ladite activation élevée d'ostéoclastes étant associée à la présence d'autoanticorps chez ledit sujet, ledit procédé et/ou kit comprenant l'un ou plusieurs parmi les suivants :
- un dosage pour déterminer le niveau d'activation d'ostéoclastes,
- un dosage pour déterminer la présence et l'identité d'autoanticorps, y compris la présence d'anticorps à des antigènes citrullinés, et
- un questionnaire pour l'évaluation de manière quantitative et éventuellement de manière qualitative d'une douleur, et en particulier d'une douleur articulaire (arthralgie), et éventuellement également
- un dosage, le moyen pour, ou une étape d'évaluation de manière qualitative ou de manière quantitative de la densité osseuse.

6. Kit pour une utilisation selon la revendication 5, comprenant en outre un dosage pour l'évaluation de manière qualitative et/ou de manière quantitative de la présence de facteurs rhumatoïdes dans un échantillon prélevé à partir dudit individu.
